(19) Europäisches Patentamt
European Patent Office
Office européen des brevets

(11) Publication number: **0 125 228**
**B1**

(12) # EUROPEAN PATENT SPECIFICATION

(45) Date of publication of patent specification: **15.06.88**

(51) Int. Cl.[4]: **C 12 N 15/00,** C 12 N 5/00

(21) Application number: **84870058.9**

(22) Date of filing: **26.04.84**

(54) **Plasmids and modified strains of Vibrio cholerae, their preparation and derived vaccines.**

(30) Priority: **29.04.83 US 489958**

(43) Date of publication of application:
**14.11.84 Bulletin 84/46**

(45) Publication of the grant of the patent:
**15.06.88 Bulletin 88/24**

(84) Designated Contracting States:
**AT BE CH DE FR GB IT LI LU NL SE**

(56) References cited:
**EP-A-0 018 154**
**EP-A-0 119 031**
**GB-A-2 032 955**
**LANCET 2, No. 8256 (1981), p. 1162-63**
**PROC. NATL. ACAD. SCI USA 79 (1982) p. 2976-80**
**NUCL. ACIDS REJ. VOL. 10, NO. 16 (1982) p. 4883-90**

(73) Proprietor: **PRESIDENT AND FELLOWS OF HARVARD COLLEGE**
**17 Quincy Street**
**Cambridge Mássachusetts 02138 (US)**

(72) Inventor: **Mekalanos, John J.**
**30 Florissant Avenue**
**Framingham Massachusetts 01701 (US)**

(74) Representative: **Tasset, Gérard**
**SMITHKLINE - RIT rue de l'Institut, 89**
**B-1330 Rixensart (BE)**

(56) References cited:
**PROCEEDINGS OF THE NATIONAL ACADEMY OF SCIENCES OF THE UNITED STATES OF AMERICA, vol. 79, no. 1, January 1982, Baltimore, USA J.J. MEKALANOS et al. "Isolation of enterotoxin structural gene deletion mutations in Vibrio/cholerae induced by two mutagenic vibriophages" pages 151-155**
**PROCEEDINGS OF THE NATIONAL ACADEMY OF SCIENCES OF THE UNITED STATES OF AMERICA, vol. 76, no. 4, April 1979, Baltimore, USA T. HONDA et al. "Selection and characteristics of a Vibrio cholerae mutant lacking the A (ADP-ribosylating) portion of the cholera enterotoxin" pages 2052-2056**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 125 228 B1**

Courier Press, Leamington Spa, England.

**Description**

The invention relates to plasmids and modified strains of *Vibrio cholerae* and to the preparation thereof. The modified strains are non-toxinogenic *Vibrio cholerae* mutants useful, for example, for immunological protection against cholera and other diseases.

Cholera is a diarrheal disease caused by *Vibrio cholerae*, a gram-negative bacterium. Orally ingested, *V. cholerae* grows in the upper intestine of man and produces a soluble protein, cholera toxin, which is responsible for the diarrhea observed in the disease. The toxin is composed of two types of subunits, A and B, and the activity of the intact toxin finds its origin in a proteolytic fragment of the A subunit, the $A_1$ peptide, which is an enzyme that activates the adenylate cyclase system of target cells [Gill (1975) Proc. Nat'l. Acad. Sci. USA *72*:2064—2068]. The resultants increase in cyclic AMP in intenstinal cells causes the diarrhea seen in the disease. The B subunit is nontoxic, although it does bind to the target cell and facilitate the transport of the $A_1$ peptide through the cell's membrane [Cuatrecasas, Biochem. *12*:3577—3581 (1973)]. Antibodies directed at the B subunit efficiently inactivate the toxin by blocking the binding of the toxin to the cell surface (*Id*].

Because cholera toxin is an intestinal disease, commercially available killed bacterial and toxoid vaccines have been relatively ineffective in inducing immunity when administered parenterally. Since the disease itself induces immunity, one must conclude that local intestinal immunity mediated by secretory IgA is probably the most important aspect of acquired immunity to cholera. To stimulate local immunity in the intestine, the bacterial antigens must be delivered through the acid and proteolytic barrier of the stomach. For this reason, live, oral cholera vaccines have been proposed.

Mutants of *V. cholerae* have been isolated which produce no toxin or produce only the B subunit of the toxin [Finkelstein et al., (1974) J. Infect. Dis. *129*:117—123; Honda et al., (1979) Proc. Nat'l. Acad. Sci. USA *76*: 2052—2056; Mekalanos et al., (1982) Proc. Nat'l. Acad. Sci. USA *79*:151—155]. However, to be an optimum candidate for a live anti-cholera vaccine, a mutant should: 1) be well-characterized and genetically stable (i.e., it should not revert to the toxin producing wild-type); 2) colonize well in the intestine and 3) provide long-lived, broad-based immunity. Mutants produced thus far, for example, those produced by chemical mutagenesis or those produced from parent strains not known to have optimal colonization and immunity-producing ability may pose problems in one of the three areas listed above, even though preliminary testing in human volunteers shows they are relatively innocuous and they induce significant immunity [Woodward et al., (1975), Proc. 11th Jt. Conf. on Cholera NIH, p. 330; Holme et al., *Acute Enteric Infections in Children, New Prospects for Treatment and Prevention* (1981) Elsevier/North-Holland Biomedical Press, Ch. 26, pp. 443 et seq. (Levine et al.].

In The Lancet, vol. 2, No. 8256 (1981), p. 1162—3, J. B. Kaper and M. M. Levine reported that "deletion mutants of the toxin region have been prepared which could be introduced into fully pathogenic strains of *V. cholerae* to render them non-toxinogenic without affecting other vibrio functions such as the ability to colonise the small intestine". Nevertheless, this paper provide no indication regarding the process to be used for obtaining such deletation mutants and there is no evidence that the toxin sequences were localized on the cloned HindIII fragment. The authors state that probes specific for A and B subunits of CT were used to confirm the work previously carried out with LT and indicate a figure which, however, gives no indication that they had indeed subcloned and identified the A and B subunit sequences. The probe is simply an otherwise unspecified cloned fragment of CT DNA and there is therefore no evidence or suggestion that the A and B sequences were indeed identified in order to introduce deletions. Further, the paper gives no indication of how to reintroduce deleted toxin sequences or which recipient strain would be adequate for the introduction and the only evidence is that they cloned from an E1 Tor strain.

The invention features, in one aspect, a genetically stable, nontoxinogenic form of the Ogawa 395 strain of *Vibrio cholerae* which has a deletion mutation in both copies of the $A_1$-peptide-encoding gene (*ctx*A) resulting in the loss of a gene sequence required for expression of a toxic $A_1$ peptide.

In preferred embodiments, each copy of the *ctx*A gene sequence is missing at least the portion of the gene sequence which codes for amino acids 41 through 101 (and most preferably, for amino acids 10 through 164) of the $A_1$ subunit; either the *ctx* B gene sequence is intact or the deletion mutation includes a portion of the B-subunit encoding gene required for expression of an effective B-subunit; and the *V. cholerae* cell line has the identifying characteristics of the deposits made with the American Type Culture Collection under ATCC Nos. 39,346 or 39,347.

In other aspects, the invention features a method of making a genetically stable form of *V. cholerae* that is incapable of expressing a toxic $A_1$ peptide and a plasmid used in that method.

In preferred embodiments, the method includes the steps of isolating a first plasmid containing the *ctx* A and *ctx* B genes and DNA segments bracketing both sides of those genes; constructing a second plasmid from the first plasmid, in which a sequence in the $A_1$-peptide-encoding gene required for expression of a toxinogenic $A_1$ peptide is deleted; recombining a segment of the second plasmid which includes the bracketing gene segments onto the chromosome of a *V. cholerae* strain known to colonize the intestine and to be immunogenic, thus replacing one copy of the *ctx*A gene on the chromosome with the deletion mutation and growing the resulting microorganism for several generations to allow spontaneous transfer of the mutation onto the other copy of the *ctx* A gene. Also in preferred embodiments of the method, the second plasmid includes a genetic marker to verify the recombination step, the strain used for the recombination step is Ogawa 395, and the deletion involved is as described above.

The plasmid comprises a DNA sequence corresponding to the segment of the chromosome which brackets the *ctx* A-*ctx* B gene of a strain of *V. cholerae* and the plasmid lacks at least a segment of the *ctx* A gene required for expressing a toxic $A_1$ peptide.

Other features and advantages of the invention are apparent from the following description of the preferred embodiments and the claims.

Description of the preferred embodiments

In the drawings which illustrate the preferred embodiments:

Fig. 1 is a diagram representing the steps of the method of creating a plasmid to be recombined onto the chromosome of *V. cholerae*.

Figs. 2A and B are diagrams representing the steps for recombination of plasmids from Fig. 1 onto the chromosome of *V. cholerae*.

Fig. 3 is a copy of a photograph of a Southern blot analysis.

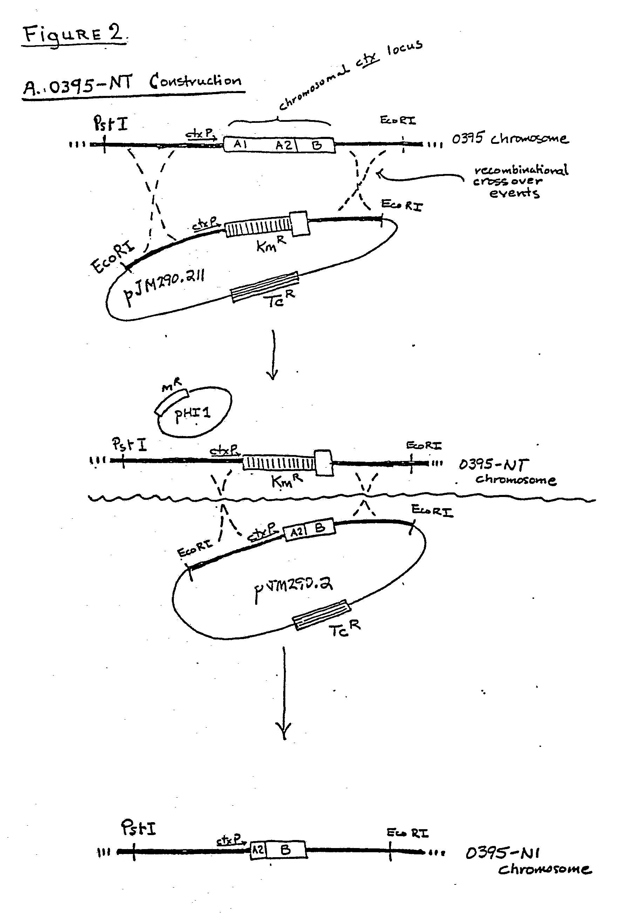

The following abbreviations apply to Figs. 1 and 2.

| Gene sequence | Resistance markers |
|---|---|
| *ctx*p=toxin promoter | Gm=gentomycin |
| $A_1$=$A_1$ subunit promoter | Tc=tetracycline |
| $A_2$=$A_2$ subunit sequence | Km=kanamycin |
| B=B sequence | Rep=origin of plasmid replication |

Conditions used for digestion of the plasmids with the indicated restriction enzymes were those suggested by the vendor, New England Biolabs, Beverly, MA, U.S.A.. Similarly, T4 DNA polymerase, Nuclease Bal-31, Klenow fragment and DNA ligase were used as suggested by their manufacturer, Bethesda Research Labs., Inc., Bethesda, MD, U.S.A.. XbaI and EcoR1 linkers were purchased from New England Biolabs. The open boxes labelled A1, A2, and B represent the corresponding regions of the *ctx*A and *ctx*B genes. The heavy lines show *V. cholerae* DNA which is adjacent to the toxin genes.

I. Selecting the parental strain

The parental strain of *V. cholerae* used to construct a mutant for live vaccines should induce longlasting, comprehensive protection and should colonize well in the human intestine. Ogawa 395 has demonstrated an ability to induce an immunity lasting three years which is generally effective against infection by other strains [Cash et al., (1974) J. Infect. Dis. *129*:45—52]. It also colonizes well in the intestine.

II. Constructing the plasmid

In broad outline, a plasmid (pJM17) prepared from DNA of wild-type Inaba 569B *V. cholerae* according to the procedure of Pearson and Mekalanos (1982) Proc. Nat'l. Sci. USA *79*:2976—2980 contains genes for the A toxin subunit (*ctx*A) and the B toxin subunit (*ctx*B), as well as for tetracycline resistance ($Tc^R$).

As shown in Fig. 1, the pJM17 plasmid was linearized with *Xba*I and then exonucleolytically digested with *Bal*31. Since the *Xba*I site resides within the *ctx*A gene, this procedure resulted in deletion of *ctx*A sequences. Ligation of the *Bal*-31 digests in the presence of an *Xba*I DNA linker resulted in the construction and isolation of plasmid pJM17.23. Reassortment of the *Pst*I-*Xba*I fragments of pJM17 and pJM17.23 resulted in construction of pJM23. This plasmid contains an internal 450 base pair deletion in *ctx*A. The position of this deletion within the *ctx*A gene was confirmed by DNA sequencing which showed that over 80% of the sequence required for production of the $A_1$ peptide was deleted. Specifically, the *ctx*A deletion carried by pJM23 removes the DNA encoding amino acid residues 10 through 164. Since the enzymatically active $A_1$ peptide is 192 amino acid residues in length, this deletion removes over 80% of the required information for $A_1$. The DNA sequence of the *ctx*A deletion is shown below:

```
                <--------------------------------------------|
AGT AAC TTA GAT ATT GCT CCA GCA GCA GAT GGT TAT GGA T|TG GCA GGT TTC CCT CCG GAG CAT AGA GCT TGG
                                                     |
SER ASM LEU ASP ILE ALA PRO ALA ALA ASP GLY TYR GLY L|EU ALA GLY PHE PRO PRO GLU HIS ARG ALA TRP
                155                 160              |     165                 170
                                                     |
(continues to residue 10)<---------------------------|
```

It has been reported that a 12,500 dalton molecular weight fragment of $A_1$ peptide, which contains the region from MET-41 to MET-101, retains 35% of the enzymatic activity of a complete $A_1$ peptide [Lai et al.

(1979) Abstracts of the 11th International Congress of Biochemistry, Toronto, Canada, p. 207, Abstract 03-45173]. The active site of the $A_1$ peptide thus lies on that 12,500-dalton fragment and, even if it is not within the 6600-dalton region between MET-41 and MET101, it cannot lie more than about 54 amino acids (5900 daltons) past the MET-101. The active site is thus well within the *ctx*A deletion of plasmid pJM23. Deletion of the amino acids between amino acid 41 and 101 (or most preferably, to ensure total absence of toxinogenicity between amino acids 10 and 164) assures lack of $A_1$ toxinogenic activity.

Plasmid pJM23 is shown to code for production of B subunit in both *E. coli* and in *V. cholerae*, thus showing that deletion mutations in *ctx*A have little effect on expression of *ctx*B. The following results (Table 1) were obtained by assaying total B subunit produced in 18 Hr CYE cultures by enzyme linked immunosorbant assay. Less than 0.005 μg/ml of B subunit could not be detected. ND means not determined because the experiment was prohibited.

TABLE 1
B Subunit production by recombinant plasmids

| Plasmid | In *E. coli* MS371 | In *V. cholerae* M7922 |
|---|---|---|
| pJM17 | 0.3 μg/ml | ND |
| pJM23 | 0.2 μg/ml | 9.0 μg/ml |
| pJM290.2 | 0.05 μg/ml | 1.5 μg/ml |
| pJM23.211 | <0.005 μg/ml | <0.005 μg/ml |
| pJM290.211 | <0.005 μg/ml | <0.005 μg/ml |

III. Recombination onto the chromosome

Referring again to Fig. 1, two plasmids, pJM23.2 and pJM23.211, are constructed from pJM23. Fragments of those plasmids are then cloned onto another plasmid (RK290) [Runken et al. (1981) Nature (London) *289*:85—88] for use in marker exchanges depicted in Fig. 2 and described below.

A. Strain 0395-N1 from plasmid pJM23

As shown in Fig. 1, pJM23 is converted to a derivative plasmid pJM23.2 by digestion with PstI and $T_4$ polymerase followed by ligation in the presence of EcoRI linker. pJM23.2 is identical to pJM23, except that the PstI site has been replaced by a EcoRI site. From pJM23.2 a plasmid (pJM23.211) is constructed with a DNA fragment encoding for resistance to kanamycin ($Km^R$) inserted approximately at the junction of the *ctx*A to *ctx*B deletion originally on pJM23.

This is accomplished by digesting pJM23.2 with XbaI in the presence of the XbaI products of plasmid pJM22 carrying $Km^R$ flanked by XbaI/HincII sites. The resulting plasmid pJM23.21 is digested with HincII and ligated to create pJM23.211, which has a *ctx*A deletion starting at amino acid 10 and extending through *ctx*B to the HincII site.

The $Km^R$ gene provides a selective marker with which to obtain the recombinants of 0395 which have replaced both their resident *ctx*A genes with the deletion mutation carried by pJM23.211.

A marker exchange procedure is then used to effect recombination onto the chromosome, as follows: A spontaneous streptomycin resistant ($Sm^R$) derivative of 0395 is first made by plating out about $10^{10}$ Ogawa 0395 cells on medium containing 100 μg/ml of streptomycin. This derivative is used in all subsequent experiments. Plasmid pJM290.211 is transferred to 0395 $Sm^R$ by conjugation using plasmid RK2013 [Ruvkin et al. (1981) Nature (London) *289*:85—88] as a mobilizer. 0395 $Sm^R$ cells carrying pJM290.211 are $Sm^RTc^RKm^R$.

These are superinfected with another plasmid pHI1 [pHI1 is the same as plasmid pHI1J1, reported by Berlinger et al. (1978) Nature *276*:633—634], which is $Gm^R$, and $Gm^RKm^R$ colonies are selected. pHI1 and pJM290.211 cannot stably coexist in the same cell because of an incompatibility barrier. Thus, in order for the cell to remain $Km^R$, it must recombine the DNA coding for this resistance onto the 0395 chromosome.

Fig. 2A shows the crossover events between a chromosomal copy of the *ctx* genes and the deletion derivative carried by plasmid pJM290.211. The appropriate recombinants were recovered by superinfecting with incompatible plasmid pHI1 and selecting $Gm^RKm^R$. The structure of such a recombinant, 0395-NT, is shown. The site on the 0395 chromosome where the $Km^R$ will end up is determined by the DNA sequences flanking the $Km^R$ fragment. Since the flanking sequences are derived from DNA which flanks the toxin genes on the chromosome, the result shown in Figure 2A occurs, and the resident genes are replaced by the mutant genes carrying the $Km^R$ insert. The extent of the DNA on pJM23.211 which brackets the *ctx*A and *ctx*B gene sites and corresponds to the wild-type chromosomal DNA sequence can be expressed as the distance between the EcoRI site and the XbaI site in the A gene fragment on pJM23.21 (2.7 kilobases) and the distance between the HincII site in the B gene, and the EcoRI

site adjacent the $Tc^R$ gene (1.4 kilobases). Thus, the deletion mutation is recombined onto the chromosome and replaces one normal copy of the *ctx* locus.

Since 0395 has two copies of the toxin locus, it is necessary to recombine the 290.211 deletion mutation into both of these. Surprisingly, this is simply accomplished by growing strains carrying one marker exchanged copy for several generations during which time recombination events resulted in the spontaneous transfer of the mutation into the other copy. 0395 derivatives carrying the deletion mutations in both copies are then recognized by colony hybridization using a probe (the 0.95 Kb XbaI/HincII fragment of pJM17) which specifically hybridizes to the DNA absent in the deletion mutation. Colonies unreactive with this probe are then checked by Southern hybridization to confirm the loss of the sequence removed by the deletion. The migration of the bands confirms that the expected size deletions of the A gene are introduced into each of the two resident *ctx* gene copies of 0395 to give strain 0395-NT.

As expected for a *ctx*A-*ctx*B deletion; strain 0395-NT produces neither the A nor the B subunit since portions of both these genes were deleted on pJM290.211. The results below (Table 2) show total B subunit antigen produced in 18 Hr CYE cultures and assayed by enzyme linked immunosorbant assay. Less than 0.005 μg/ml of B subunit could not be detected. Total toxin activity expressed in terms of μg/ml of purified cholera toxin. The same culture supernatant fluid was assayed as above for B subunit. The toxicity assay was the CHO-cell assay performed as described by Guerrant et al. (1974) Infet. Imm. *10*:320—327.

TABLE 2
B Subunit and toxin activity produced by *V. cholerae* derivatives

| Strain | B Subunit antigen | Toxin activity |
|---|---|---|
| 0395 | 0.3 μg/ml | 0.3 μg/ml |
| 0395-NT | <0.005 μg/ml | <0.001 μg/ml |
| 0395-N1 | 0.3 μg/ml | <0.001 μg/ml |

B. Strain 0395-N1

The construction present on pJM23.2 is crossed onto the chromosome by a modified marker exchange procedure. Plasmid pJM290.2 is formed by transferring a 4.6 kilobase pair fragment from pJM23.2 onto RK290. Plasmid pJM290.2 is then transferred into strain 0395-NT which has each of its two *ctx* copies tagged with a $Km^R$. Introduction of pJM290.2 into this strain then allowed crossover events to subsequently replace the $Km^R$-tagged copies with the construction on pJM290.2. These recombinant colonies were recognized by their sensitivity to Km.

Specifically, as shown in Fig. 2B strain 0395-NT was cured of pHI1 and then pJM290.2 was transferred into the strain. After about 50 generations of growth, spontaneous $Km^S$ recombinants were isolated by replica plating. One of these was cured of pJM290.2 and has the structure shown (0395-N1). Southern hybridization to confirm the structure of one of these to give strain 0395 N1 is done as follows:

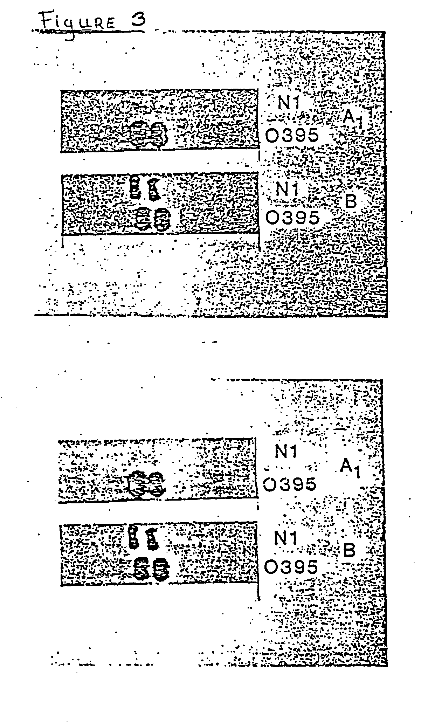

DNA is prepared from strains 0395 and 0395-N1. The DNA is digested with *Xba*I and analyzed by the method of Southern (1975) J. Molec. Biol. *98*:503—517 with either an A1 probe (the 580 base pair XbaI-HindIII fragment of EWD299 [Dallas (1979) J. Bact. *139*:850—858] or B probe (the 590 base pair EcoRI-HindIII fragment of EWD299). The two *ctx* loci of 0395 are seen as two bands which react with both probes. Strain 0395-N1, however, reacts only with the B probe and the bands which do react are smaller in size by the *ctx*A deletion originally present on pJM23. Fig. 3 is a copy of a photograph of blots from such a Southern hybridization showing that 0395-N1 has lost the sequence homologous to a probe derived from the $A_1$ region but retains the sequence for the B subunit which reside on a restriction fragments that are smaller by the size of the *ctx*$A_1$ deletion on plasmid pJM23 (450 base pairs). Consistent with this result, 0395-N1 produced normal amounts of the B subunit when compared to the 0395 parental strain but displayed no toxicity in CHO cells (Table 2, above). Thus, 0395-N1 is identical to the parental strain 0395 except for the *ctx*A deletion originally present on pJM23.

The resulting strains and plasmids have been deposited with the American Type Culture Collection and have the following deposit numbers:

| | |
|---|---|
| Strain 0395-N1 | ATCC 39,346 |
| Strain 0395-NT | ATCC 39,347 |
| Plasmid pJM290.2 | ATCC 39,348 |
| Plasmid pJM290.211 | ATCC 39,349 |

The ATCC deposit samples will be freely available upon issuance of a United States patent hereon,

upon publication of a European or Japanese application hereon, or in accordance with the laws of any country in which a patent application is filed.

IV. Use Both strain 0395-N1 and strain 0395-NT are useful as sources of immunological protection against cholera infection. They are useful for both live and dead vaccines.

Strain 0395-N1 is produced from a background strain that is known to colonize well in the intestine and to provoke a strong immunity reaction. The mutant strain has been demonstrated to be genetically altered only insofar as it has a deletion mutation which prevents the expression of a toxinogenic $A_1$ toxin subunit. The strain is useful, inter alia, as a vaccine prepared and administered under the dosages and conditions described in Holme et al., *Acute Enteric Infections in Children, New Prospects for Treatment and Prevention* (1981) Elsevier/North Holland Biomedical Press, Ch. 26, pp. 443 et seq. (Levine et al.).

The fact that individuals infected with toxinogenic *V. cholerae* Ogawa 395 do not excrete significant quantities of intact cells indicates that a cell-targeted immunological defense in addition to anti-toxin defenses are involved. Strain 0395-NT, while lacking genetic information to produce the B-subunit of the toxin, nevertheless possesses other immunogenic traits of the Ogawa 395 and is useful as a vaccine prepared and administered as described above.

The plasmids and methods described are useful for making 0395-NT and 0395-N1 strains and may also be used for making strains which express the B subunit with some other antigen. Such strains could then be used in a vaccine to take advantage of the improved immunogenicity realized by attaching an antigen to the B subunit and thus using the B subunit as a cell recognition and attachment vehicle.

**Claims for the Contracting States: BE, CH, DE, FR, GB, IT, LI, LU, NL, SE**

1. A non toxinogenic genetically stable form of the Ogawa 395 strain of *Vibrio cholerae* which has a deletion mutation in both copies of the $A_1$ peptide-encoding gene (*ctx*$A_1$) resulting in the loss of a gene sequence required for expression of a toxic $A_1$ peptide.

2. The strain of claim 1 wherein each copy of the *ctx*$A_1$ gene is missing at least that portion of the gene sequence which codes for amino acid residues 41 through 101.

3. The strain of claim 1 wherein each copy of the *ctx*$A_1$ gene is missing at least that portion of the gene sequence which codes for amino acid residues 10 through 164.

4. The strain of claim 1 wherein each copy of said *ctx*$A_1$ gene lacks at least 80% of the sequence required for expression of the $A_1$ peptide.

5. The strain of claim 1 wherein the *ctx*B gene sequence is intact.

6. The strain of claim 1 wherein the wild-type genetic sequence is otherwise intact, except for said deletion mutation.

7. The strian of claim 1 wherein said deletion mutation includes a portion of the B subunit encoding gene (*ctx*B) required for expression of an effective B subunit.

8. A cell line according to claim 1 which is either of the cell lines having ATCC Deposit No. 39,346 or ATCC deposit No. 39,347.

9. A method of producing a genetically stable form of *Vibrio cholerae* that is incapable of expressing a toxic $A_1$ peptide, comprising

providing a wild-type strain of *Vibrio cholerae* having at least two copies of the *ctx*A gene on its chromosome and which is known to colonize in the intestine and to be immunogenic; causing a deletion mutation on one copy of the *ctx*A gene of a DNA sequence of said strain required for expression of a toxic $A_1$ peptide and growing the resulting altered *V. cholerae* for several generations to allow spontaneous transfer of the alteration to the second copy of the *ctx*A gene on said chromosome.

10. The method of claim 9 wherein said deletion step comprises the steps of:

providing DNA from a wild-type strain of *V. cholerae*; isolating from that strain a first plasmid which contains the *ctx*A and *ctx*B genes and DNA segments bracketing both sides of those genes of said strain; constructing a second plasmid from the first plasmid, the second plasmid being altered in that at least a segment of the *ctx*A gene required for expression of a toxic $A_1$ peptide has been deleted and recombining a segment of the second plasmid which includes said bracketing DNA segments onto the chromosome of a *V. cholerae* strain known to colonize in the intestine and to be immunogenic, thus replacing one copy of the *ctx*A gene on said chromosome.

11. The method of claim 10 wherein said second plasmid includes a marker to verify said recombination step.

12. The method of claim 10 wherein said strain used for recombination with said second plasmid is Ogawa 395.

13. The method of claim 10 wherein the segment deleted from said *ctx*A gene comprises at least that portion of the gene sequence which codes for amino acid residues 41 through 101.

14. The method of claim 10 wherein the segment deleted from said *ctx*A gene comprises at least that portion of the gene sequence which codes for amino acid residues 10 through 164.

15. The method of claim 10 wherein the segment deleted from said *ctx*A gene comprises at least 80% of the sequence required for expression of the $A_1$ peptide.

16. A plasmid comprising a DNA sequence corresponding to a segment of the chromosome which brackets the *ctx*A-*ctx*B gene of a strain of *V. cholerae* wherein said plasmid lacks at least a segment of the *ctx*A gene required for expression a toxic $A_1$ peptide, which is a plasmid selected from the group comprising ATCC Deposit Nos. 39,348 and 39,349.

**Claims for the Contracting State: AT**

1. A method of producing a genetically stable form of *Vibrio cholerae* that is incapable of expressing a toxic $A_1$ peptide, comprising

providing a wild-type strain of *Vibrio cholerae* having at least two copies of the *ctx*A gene on its chromosome and which is known to colonize in the intestine and to be immunogenic; causing a deletion mutation on one copy of the *ctx*A gene of a DNA sequence of said strain required for expression of a toxic $A_1$ peptide and growing the resulting altered *V. cholerae* for several generations to allow spontaneous transfer of the alteration to the second copy of the *ctx*A gene on said chromosome.

2. The method of claim 1 wherein said deletion step comprises the steps of:

providing DNA from a wild-type strain of *Vibrio cholerae*; isolating from that strain a first plasmid which contains the *ctx*A and *ctx*B genes and DNA segments bracketing both sides of those genes of said strain; constructing a second plasmid from the first plasmid, the second plasmid being altered in that at least a segment of the *ctx*A gene required for expression of a toxic $A_1$ peptide has been deleted and recombining a segment of the second plasmid which includes said bracketing DNA segment onto the chromosome of a *V. cholerae* strain known to colonize in the intestine and to be immunogenic, thus replacing one copy of the *ctx*A gene on said chromosome.

3. The method of claim 2 wherein said second plasmid includes a marker to verify said recombination step.

4. The method of claim 2 wherein said strain used for recombination with second plasmid is Ogawa 395.

5. The method of claim 2 wherein the segment deleted from said *ctx*A gene comprises at least that portion of the gene sequence which codes for amino acid residues 41 through 101.

6. The method of claim 2 wherein the segment deleted from said *ctx*A gene comprises at least that portion of the gene sequence which codes for amino acid residues 10 through 164.

7. The method of claim 2 wherein the segment deleted from said *ctx*A gene comprises at least 80% of the sequence required for expression of the $A_1$ peptide.

**Patentansprüche für die Vertragsstaaten: BE, CH, DE, FR, GB, IT, LI, LU, NL, SE**

1. Nicht toxinogene, genetisch stabile Form des Stammes Ogawa 395 von *Vibrio cholerae*, die eine Deletionsmutation in beiden Kopien des $A_1$-Peptid-codierenden Gens (*ctx*$A_1$) aufweist, die zum Verlust einer für die Expression eines toxischen $A_1$-Peptids notwendigen Gensequenz führt.

2. Stamm nach Anspruch 1, in dem jeder Kopie des *ctx*$A_1$ Gens zumindest der Teil der Gensequenz fehlt, der für die Aminosäure reste 41 bis 101 codiert.

3. Stamm nach Anspruch 1, in dem jeder Kopie des *ctx*$A_1$-Gens zumindest der Teil der Gensequenz fehlt, der für die Aminosäurereste 10 bis 164 codiert.

4. Stamm nach Anspruch 1, in dem jeder Kopie des *ctx*$A_1$-Gens zumindest 80% der für die Expression des $A_1$-Peptids notwendigen Sequenz fehlen.

5. Stamm nach Anspruch 1, in dem die *ctx*B-Gensequenz intakt ist.

6. Stamm nach Anspruch 1, in dem die Wildtyp-Gensequenz mit Ausnahme der Deletionsmutation intakt ist.

7. Stamm nach Anspruch 1, in dem die Deletionsmutation einen Teil des für die B-Untereinheit codierenden Gens (*ctx*B) umfaßt, der für die Expression einer wirksamen B-Untereinheit nötig ist.

8. Zellinie nach Anspruch 1, die entweder unter ATCC 39,346 oder ATCC 39,347 hinterlegt ist.

9. Verfahren zur Herstellung einer genetisch stabilen Form von *Vibrio cholerae*, die unfähig zur Expression eines toxischen $A_1$-Peptids ist, bei dem man einen auf seinem Chromosom zumindest zwei Kopien des *ctx*A-Gens enthaltenden Wildtypstamm von *Vibrio cholerae* bereitstellt, der bekanntermaßen im Darm Kolonien bildet und immunogen ist; eine Deletionsmutation in einer Kopie des *ctx*A-Gens jener DNA-Sequenz des Stammes durchführt, die notwendig ist für die Expression eines toxischen $A_1$-Peptids, und den erhaltenen, modifizierten *Vibrio cholerae* Stamm mehrere Generationen lang züchtet, um den spontanen Einbau der Modifikation in die zweite Kopie des *ctx*A-Gens auf dem Chromosom zu ermöglichen.

10. Verfahren nach Anspruch 9, bei dem der Deletionsschritt folgende Schritte umfaßt:

Bereitstellen der DNA eines Wildtypstammes von *Vibrio cholerae*; Isolieren eines ersten Plasmids aus diesem Stamm, das die *ctx*A und *ctx*B-Gene und die beide Seiten dieser Gene des Stammes flankierenden DNA-Segmente enthält;

Herstellen eines zweiten Plasmids aus dem ersten Plasmid, wobei das zweite Plasmid so verändert ist, daß zumindest jenes Segment des *ctx*A-Gens, das für die Expression des toxischen $A_1$-Peptids notwendig ist, deletiert worden ist, und Rekombinieren eines Segments des die flankierenden DNA-Sequenzen einschließenden zweiten Plasmids mit dem Chromosom eines *Vibrio cholerae* Stammes, der bekanntermaßen im Darm Kolonien bildet und immunogen ist, wodurch eine Kopie des *ctx*A-Gens im genannten Chromosom ersetzt wird.

11. Verfahren nach Anspruch 10, in dem das zweite Plasmid einen Marker zur Bestätigung des Rekombinationsschrittes umfaßt.

12. Verfahren nach Anspruch 10, in dem der für die Rekombination mit dem zweiten Plasmid verwendete Stamm Ogawa 395 ist.

13. Verfahren nach Anspruch 10, in dem das aus dem *ctx*A-Gen deletierte Segment zumindest den Teil der Gensequenz umfaßt, der für die Aminosäurereste 41 bis 101 codiert.

14. Verfahren nach Anspruch 10, in dem das aus dem *ctx*A-Gen deletierte Segment zumindest den Teil der Gensequenz umfaßt, der für die Aminosäurereste 10 bis 164 codiert.

15. Verfahren nach Anspruch 10, in dem das von dem *ctx*A-Gen deletierte Segment zumindest 80% der für die Expression des $A_1$-Peptids notwendigen Sequenz umfaßt.

16. Plasmid, das eine DNA-Sequenz umfaßt, die einem das *ctx*A-*ctx*B-Gen eines Stammes von *Vibrio cholerae* flankiernden Segment des Chromosoms entspricht, wobei dem Plasmid zumindest ein für die Expression des toxischen $A_1$-Peptids notwendiges Segment des *ctx*A-Gens fehlt und das Plasmid aus der Gruppe ATCC 39,348 und 39,349 ausgewählt ist.

**Patentansprüche für den Vertragsstaat: AT**

1. Verfahren zur Herstellung einer genetisch stabilen Form von *Vibrio cholerae*, die unfähig zur Expression eines toxischen $A_1$-Peptids ist, bei dem man einen auf seinem Chromosom zumindest zwei Kopien des *ctx*A-Gens enthaltenden Wildtypstamm von *Vibrio cholerae* bereitstellt, der bekanntermaßen im Darm Kolonien bildet und immunogen ist; eine Deletionsmutation in einer Kopie des *ctx*A-Gens jener DNA-Sequenz des Stammes bewirkt, die notwendig ist für die Expression eines toxischen $A_1$-Peptids und den erhaltenen, modifizierten *Vibrio cholerae* Stamm mehrere Generationen lang züchtet, um den spontanen Einbau der Modifikation in die zweite Kopie des *ctx*A-Gens auf dem Chromosom zu ermöglichen.

2. Verfahren nach Anspruch 1, bei dem der Deletionsschritt folgende Schritte umfaßt:
Bereitstellen der DNA eines Wildtyp-Stammes von *Vibrio cholerae*; Isolieren eines ersten Plasmids aus diesem Stamm, das die *ctx*A- und *ctx*B-Gene und die beide Seiten dieser Gene des Stammes flankierenden DNA-Segment enthält; Herstellen eines zweiten Plasmids aus dem ersten Plasmid, wobei das zweite Plasmid so verändert ist, daß zumindest jenes Segment des *ctx*A-Gens, das für die Expression des toxischen $A_1$-Peptids notwendig ist, deletiert worden ist, und Rekombinieren eines Segments des die flankierenden DNA-Segment einschließenden zweiten Plasmids mit dem Chromosom eines *Vibrio cholerae* Stammes der bekanntermaßen im Darm Kolonien bildet und immunogen ist, wodurch eine Kopie des *ctx*A-Gens im genannten Chromosom ersetzt wird.

3. Verfahren nach Anspruch 2, in dem das zweite Plasmid einen Marker zur Bestätigung des Rekombinationsschrittes umfaßt.

4. Verfahren nach Anspruch 2, in dem der für die Rekombination mit dem zweiten Plasmid verwendete Stamm Ogawa 395 ist.

5. Verfahren nach Anspruch 2, in dem das aus dem *ctx*A-Gen deletierte Segment zumindest den Teil der Gensequenz umfaßt, der für die Aminosäureeste 41 bis 101 codiert.

6. Verfahren nach Anspruch 2, in dem das aus dem *ctx*A-Gen deletierte Segment zumindest den Teil der Gensequenz umfaßt, der für die Aminosäureester 10 bis 164 codiert.

7. Verfahren nach Anspruch 2, in dem das von dem *ctx*A-Gen deletierte Segment zumindest 80% der für die Expression des $A_1$-Peptids notwendigen Sequenz umfaßt.

**Revendications pour les Etats Contractants: BE, CH, DE, FR, GB, IT, LI, LU, NL, SE**

1. Forme non toxinogène génétiquement stable de la souche Ogawa 395 de *Vibrio cholerae* qui a une mutation de délétion dans les deux copies du gène (*ctx*$A_1$) encodant le peptide $A_1$ résultant dans la perte d'une séquence du gène requise pour l'expression d'un peptide toxique $A_1$.

2. Souche de la revendication 1 dans laquelle chaque copie du gène *ctx*$A_1$ est dépourvue au moins de la portion de séquence du gène qui code pour les résidus aminés 41 à 101.

3. Souche de la revendication 1 dans laquelle chaque copie du gène *ctx*$A_1$ est dépourvue au moins de la portion de séquence du gène qui code pour les résidus acides aminés 10 à 164.

4. Souche de la revendication 1 dans laquelle chaque copie dudit gène *ctx*A est dépourvu d'au moins 80% de la séquence requise pour l'expression du peptide $A_1$.

5. Souche de la revendication 1 dans laquelle la séquence du gène *ctx*B est intacte.

6. Souche de la revendication 1 dans laquelle la séquence génétique du type sauvage est par ailleurs intacte, sauf en ce qui concerne ladite mutation de délétion.

7. Souche de la revendication 1 dans laquelle ladite mutation de délétion inclut une portion du gène (*ctx*B) encodant la sous unité B requise pour l'expression d'une sous unité B effective.

8. Lignée cellulaire suivant la revendication 1 qui est l'une quelconque des lignées cellulaires déposées sous les numéros ATCC 39346 et 39347.

9. Méthode pour la production d'une forme génétiquement stable de *Vibrio cholerae* qui est incapable d'exprimer un peptide toxique $A_1$ caractérisée en ce que on utilise une souche sauvage de *Vibrio cholerae* qui possède au moins deux copies du gène *ctx*A sur son chromosome et qui est connue pour coloniser dans l'intestin et être immunogène; on cause une mutation de délétion sur une copie du gène *ctx*A d'une séquence d'ADN de ladite souche et qui est requise pour l'expression d'un peptide toxique $A_1$ et on cultive le *V. cholerae* altéré résultant pendant plusieurs générations pour permettre un transfert spontané de l'altération sur la seconde copie du gène *ctx*A sur ledit chromosome.

10. Méthode de la revendication 9 dans laquelle ladite délétion comprend les stades par lesquels on utilise de l'ADN d'une souche sauvage de *V. cholerae*, on isole de cette souche un premier plasmide qui contient les gènes *ctx*A et *ctx*B et des segments d'ADN encadrant les deux côtés de ces gènes de ladite souche; on construit un second plasmide au départ du premier plasmide, le second plasmide étant altéré en ce qu'au moins un segment du gène *ctx*A requis pour l'expression d'un peptide toxique $A_1$ a été délété et on recombine un segment du second plasmide qui inclut lesdits segments d'ADN encadrant sur le chromosome d'une souche de *V. cholerae* connue pour coloniser dans l'intestin et pour être immunogène, remplaçant donc une copie du gène *ctx*A sur ledit chromosome.

11. Méthode de la revendication 10 dans laquelle ledit second plasmide inclut un marqueur pour vérifier ladite étape de combinaison.

12. Méthode de la revendication 10 dans laquelle ladite souche utilisée pour la recombinaison avec ledit second plasmide est Ogawa 395.

13. Méthode de la revendication 10 dans laquelle le segment délété dudit gène *ctx*A comprend au moins la portion de la séquence du gène qui code pour les résidus acides aminés 41 à 101.

14. Méthode de la revendication 10 dans laquelle le segment délété dudit gène *ctx*A comprend au moins la portion de la séquence du gène qui code pour les résidus acides aminés 10 à 164.

15. Méthode de la revendication 10 dans laquelle le segment délété dudit gène *ctx*A comprend au moins 80% de la séquence requise pour l'expression du peptide $A_1$.

16. Plasmide comprenant une séquence d'ADN correspondant à un segment du chromosome qui encadre le gène *ctx*A-*ctx*B d'une souche de *V. cholerae* dans laquelle ledit plasmide est dépourvu d'au moins un segment du gène *ctx*A requis pour l'expression d'un peptide toxique $A_1$ qui est un plasmide choisi dans le groupe comprenant les dépôts ATCC Nos. 39348 et 39349.

**Revendication pour le Pays Contractant: AT**

1. Méthode pour la production d'une forme génétiquement stable de *Vibrio cholerae* qui est incapable d'exprimer un peptide toxique $A_1$, caractérisée en ce que on utilise une souche sauvage de *Vibrio cholerae* qui possède au moins deux copies du gène *ctx*A sur son chromosome et qui est connue pour coloniser dans l'intestin et être immunogène; on cause une mutation de délétion sur une copie du gène *ctx*A d'une séquence d'ADN de ladite souche et qui est requise pour l'expression d'un peptide toxique $A_1$ et on cultive le *V. cholerae* altéré résultant pendant plusieurs générations pour permettre un transfert spontané de l'altération sur la seconde copie du gène *ctx*A sur ledit chromosome.

2. Méthode de la revendication 1 dans laquelle ladite délétion comprend les stades par lesquels on utilise de l'ADN d'une souche sauvage de *Vibrio cholerae*; on isole de cette souche un premier plasmide qui contient les gènes *ctx*A et *ctx*B et des segments d'ADN encadrant les deux côtés de ces gènes de ladite souche; on construit un second plasmide au départ du premier plasmide, le second plasmide étant altéré en ce que au moins un segment du gène *ctx*A requis pour l'expression d'un peptide toxique $A_1$ a été délété et on recombine un segment du second plasmide qui inclut lesdits segments d'ADN encadrant sur le chromosome d'une souche de *V. cholerae* connue pour coloniser dans l'intestin et pour être immunogène, remplaçant donc une copie du gène *ctx*A sur ledit chromosome.

3. Méthode de la revendication 2 dans laquelle le second plasmide inclut un marqueur pour vérifier ladite étape de recombinaison.

4. Méthode de la revendication 2 dans laquelle ladite souche utilisée pour la recombinaison avec ledit second plasmide est Ogawa 395.

5. Méthode de la revendication 2 dans laquelle le segment délété dudit gène *ctx*A comprend au moins la portion de la séquence du gène qui code pour les résidus acides aminés 41 à 101.

6. Méthode de la revendication 2 dans laquelle le segment délété dudit gène *ctx*A comprend au moins la portion de la séquence du gène qui code pour les résidus acides aminés 10 à 164.

7. Méthode de la revendication 2 dans laquelle le segment délété dudit gène *ctx*A comprend au moins 80% de la séquence requise pour l'expression du peptide $A_1$.

Figure 1

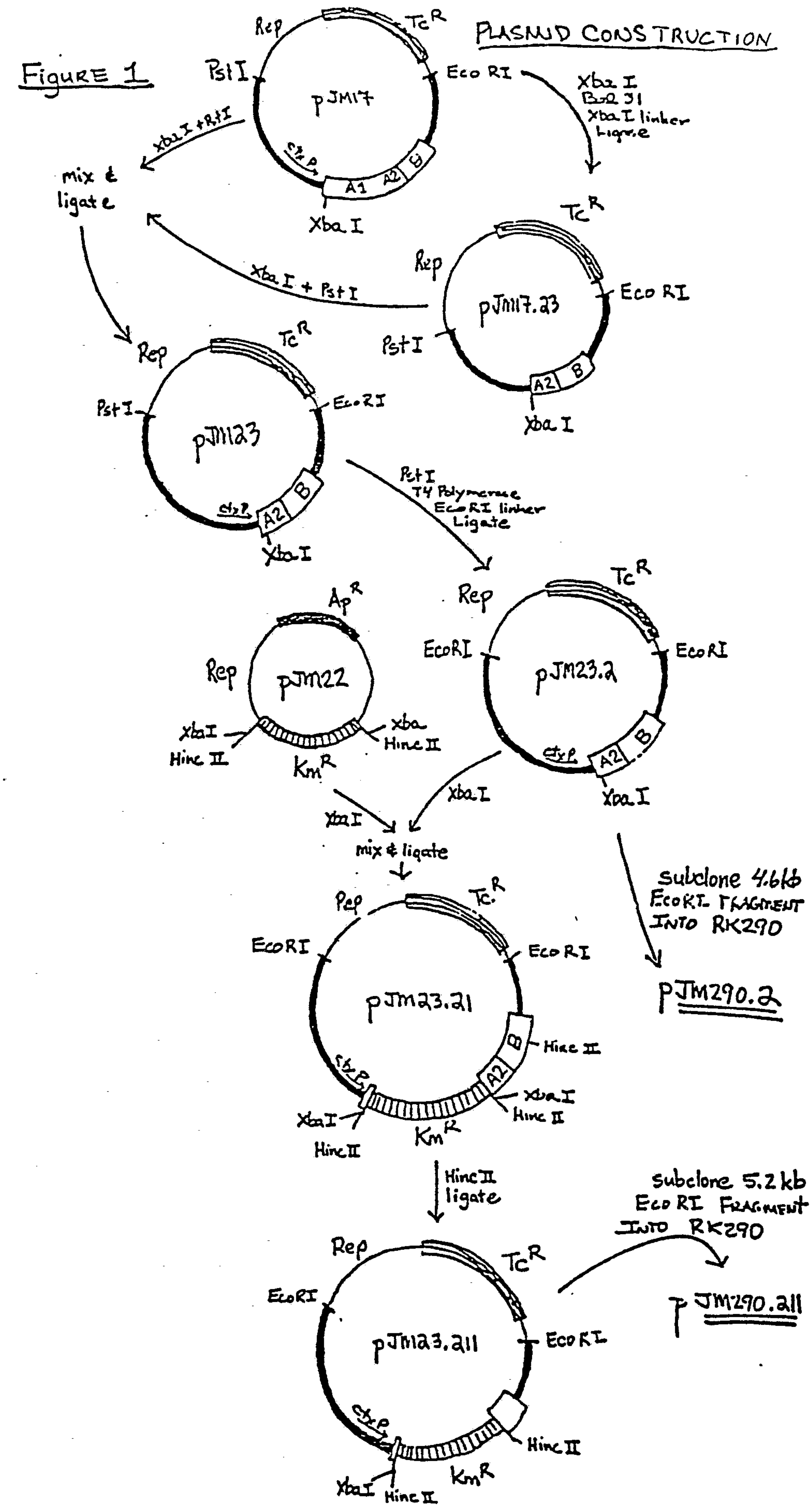
PLASMID CONSTRUCTION
pJM17
Rep
TcR
PstI
EcoRI
A1
A2
B
XbaI
XbaI + PstI
mix & ligate
XbaI
Bal 31
XbaI linker
Ligate
pJM17.23
pJM123
PstI
T4 Polymerase
EcoRI linker
Ligate
pJM22
ApR
KmR
HincII
pJM23.2
subclone 4.6kb
EcoRI FRAGMENT
INTO RK290
pJM290.2
pJM23.21
HincII
ligate
pJM23.211
subclone 5.2 kb
EcoRI FRAGMENT
INTO RK290
pJM290.211

Figure 2.

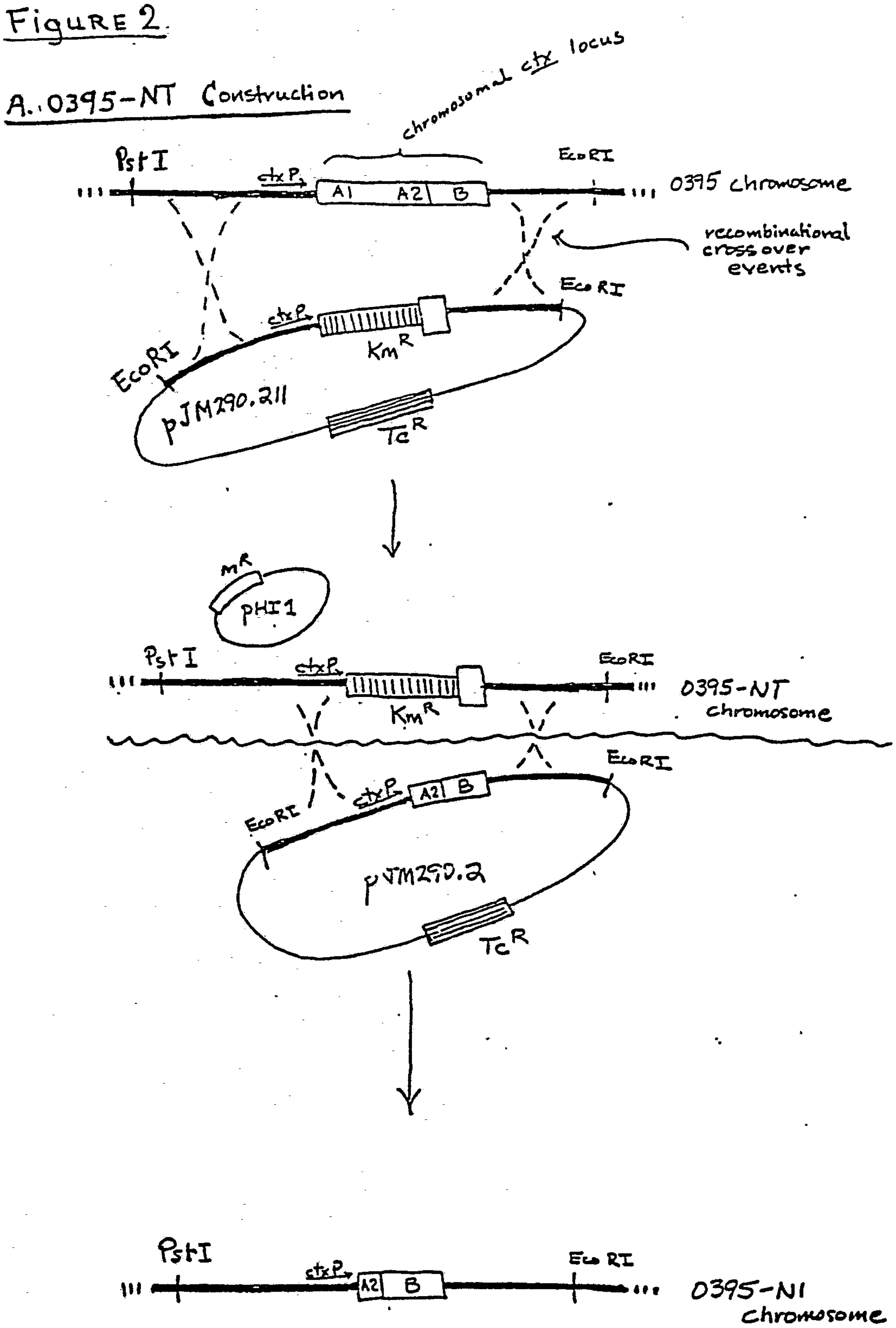
A. 0395-NT Construction
chromosomal ctx locus
PstI
ctxP
A1
A2
B
EcoRI
0395 chromosome
recombinational cross over events
EcoRI
ctxP
KmR
EcoRI
pJM290.211
TcR
mR
pHI1
PstI
ctxP
EcoRI
0395-NT chromosome
KmR
EcoRI
ctxP
A2
B
EcoRI
pJM290.2
TcR
PstI
ctxP
A2
B
EcoRI
0395-N1 chromosome

Figure 3